# EUROPEAN PATENT APPLICATION

(11) **EP 4 019 502 A1**
(43) Date of publication of application: **29.06.2022**
(21) Application number: 20855145.7
(22) Date of filing: 04.08.2020
(51) Int. Cl.: C07D 257/02, C07D 403/12, C07D 403/14, C07F 5/00, C07F 7/00

(54) **METHOD FOR PRODUCING RADIOACTIVE METAL COMPLEX**

(30) Priority: 21.08.2019 JP 2019151480
(71) Applicant: Nihon Medi-Physics Co., Ltd, Koto-ku Tokyo, 136-0075 (JP)
(72) Inventor: IMAI, Tomoyuki, Tokyo 136-0075 (JP); KIRIU, Masato, Tokyo 136-0075 (JP); IZAWA, Akihiro, Tokyo 136-0075 (JP)
(74) Representative: Westphal, Petra
(86) International application number: PCT/JP2020/029757
(87) International publication number: WO 2021/033530

(57) **Abstract**

This method for producing a radioactive metal complex comprises a step for reacting a radioactive metal with DOTA or a ligand, which is a derivative of DOTA, in a reaction solution to form a radioactive metal complex. The reaction solution contains water, a buffer, and a water-soluble organic solvent. The radioactive metal is ⁸⁹Zr or ²²⁵Ac. The ligand may have a group linked to a peptide in the structure thereof. It is also preferable that the content of the water-soluble organic solvent contained in the reaction solution be 2-50 vol%. It is also preferable to react the radioactive metal with the ligand in the reaction solution at a temperature of 30-80°C.

## Description

### Technical Field

The present invention relates to a method for producing a radioactive metal complex.

### Background Art

Studies have been conducted on a radioactive metal complex having a radioactive metal and a ligand coordinated thereto, for the purpose of use in reagents and diagnostic agents for detection of a target molecule or pharmaceuticals for treatment of diseases. In Patent Literature 1, DOTA is used as a chelating agent to be conjugated with an antibody, and the DOTA is coordinated with a radioactive metal to label the antibody with ⁹⁰Y. Non Patent Literature 1 describes a method for forming a radioactive metal complex, the method including allowing ⁸⁹Zr as a radioactive metal to react with DOTA as a ligand in a buffer solution.

Non Patent Literature 2 describes a method for forming a radioactive metal complex, the method including allowing ⁶⁸Ga or ⁴⁴Sc to react with DOTATOC, which is a DOTA derivative and serves as a ligand, in a buffer solution.

Non Patent Literature 3 describes a method for forming a radioactive metal complex, the method including allowing ⁶⁸Ga or ⁴⁴Sc to react with DOTA in ethanol-containing physiological saline.

### Citation List

### Patent Literature

Patent Literature 1: US 2005/191239 A1

### Non-Patent Literature

Non-Patent Literature 1: Pandya et al., Chem Sci. 2017; 8 (3): 2309-14.
Non-Patent Literature 2: Eppard et al., EJNMMI Radiopharm. Chem. 2017; 1, 6.
Non-Patent Literature 3: Perez-Malo et al., Inorg. Chem. 2018, 57 (10), 6107-6117.

### Summary of Invention

However, findings of the present inventors have revealed that when a derivative in which a target molecule other than an antibody, such as a peptide, is bonded to DOTA is used as a ligand, complex formation between DOTA and a specific radioactive metal does not necessarily proceed well under the conditions disclosed in Patent Literature 1 and Non Patent Literatures 1 to 3. Such a problem arises not only in DOTA but also in a derivative similar to DOTA, such as DOTAGA.

Therefore, an object of the present invention is to provide a method for producing a radioactive metal complex with excellent efficiency in forming the complex by using DOTA, a derivative thereof, or a ligand having a structure similar to DOTA.

The present invention provides a method for producing a radioactive metal complex, the method including a step of allowing a radioactive metal to react with a ligand represented by the following formula (1) in a reaction liquid to form a radioactive metal complex, wherein
the reaction liquid contains water, a buffer, and a water-soluble organic solvent, and
the radioactive metal is ⁸⁹Zr or ²²⁵Ac, 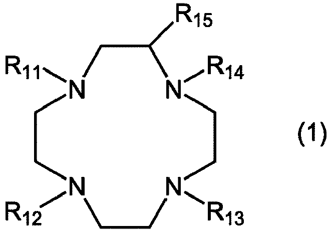
wherein R₁₁, R₁₂, and R₁₃ each independently represent a group of -(CH₂)ₚCOOH, - (CH₂)ₚC₅H₅N, -(CH₂)ₚPO₃H₂, or -(CH₂)ₚCONH₂; one of R₁₄ and R₁₅ represents a hydrogen atom or a group of -(CH₂)ₚCOOH, -(CH₂)ₚC₅H₅N, -(CH₂)ₚPO₃H₂, -(CH₂)ₚCONH₂, or - (CHCOOH)(CH₂)ₚCOOH, and the other represents a group of -(CH₂)ₚCOOH, -(CH₂)ₚC₅H₅N, - (CH₂)ₚPO₃H₂, or -(CH₂)ₚCONH₂, or a group linked to a peptide; and p represents an integer of 0 or more and 3 or less.

### Description of Embodiments

The present application claims priority to Japanese Patent Application No. 2019-151480 filed on August 21, 2019, and the entire contents of Japanese Patent Application No. 2019-151480 are incorporated herein as a part of the present specification.

The present invention can provide a method for producing a radioactive metal complex with excellent efficiency in forming the complex by using DOTA, a derivative thereof, or a ligand having a structure similar to DOTA. The present invention is particularly effective when a poorly water-soluble ligand is used.

Hereinafter, a method for producing a radioactive metal complex of the present invention will be described based on preferred embodiments thereof. The method of the present invention includes a step of allowing a radioactive metal to react with a ligand in a reaction liquid containing water, a buffer, and a water-soluble organic solvent to form a radioactive metal complex (complex forming step).

In the present step, forming a complex between the radioactive metal and the ligand is synonymous with labeling the ligand with the radioactive metal, and the efficiency in forming a complex is synonymous with a labeling ratio.

The radioactive metal in the present step is preferably used in a form of an ionizable radioactive metal compound, and more preferably used in a form of a radioactive metal ion (hereinafter, these forms are also collectively referred to as "radioactive metal source") in view of enhancing the efficiency in forming the complex. As the radioactive metal source, a liquid containing radioactive metal ions dissolved or dispersed in a solvent mainly containing water can be used, for example. A specific nuclide of the radioactive metal will be described later.

The ligand used in the present step has a structure represented by the following formula (1). That is, the ligand used in the present step is 1,4,7,10-tetraazacyclododecane-1,4,7,10-tetraacetic acid (DOTA), a derivative thereof, or a ligand having a structure similar to DOTA.

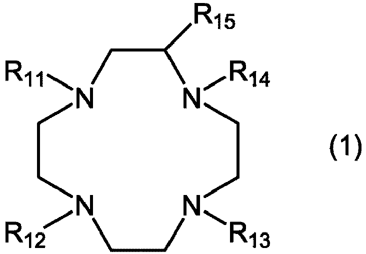

In formula (1), R₁₁, R₁₂, and R₁₃ each independently represent a group of -(CH₂)ₚCOOH, -(CH₂)ₚC₅H₅N, -(CH₂)ₚPO₃H₂, or -(CH₂)ₚCONH₂. The values of p are each independently an integer of 0 or more and 3 or less.

In formula (1), one of R₁₄ and R₁₅ represents a hydrogen atom or a group of - (CH₂)_{pCOOH}, -(CH₂)ₚC₅H₅N, -(CH₂)ₚPO₃H₂, -(CH₂)ₚCONH₂, or -(CHCOOH)(CH₂)_{pCOOH}, and the other represents a group of -(CH₂)ₚCOOH, -(CH₂)ₚC₅H₅N, -(CH₂)ₚPO₃H₂, or -(CH₂)ₚCONH₂, or a group linked to a peptide. The values of p are each independently an integer of 0 or more and 3 or less. Details of the peptide will be described later.

In the present step, when a poorly water-soluble ligand is used for forming a complex, the efficiency in forming a complex can be further enhanced. The term "poorly water-soluble" means having a property that satisfies at least one of the following conditions (i) and (ii), and preferably having a property that satisfies at least the condition (ii). The term "poorly water-soluble" also encompasses the meaning of water-insoluble, in which the ligand is not substantially dissolved in water. A case where both of the following conditions (i) and (ii) are satisfied is also encompassed by the term "poorly water-soluble".
(i) An octanol-water distribution coefficient (Log P value) of the ligand is a positive value.
(ii) An index indicating the solubility in water (Log S value) of the ligand is a negative value.

The "octanol-water distribution coefficient", which is one of indices of poor water solubility, is an index indicating hydrophobicity of a compound, and is defined as a common logarithm of a numerical value of the ratio between the distribution concentrations of a substance in each phase of a two-phase solvent system composed of n-octanol and water. The value of this common logarithm is a numerical value based on the ratio of the concentration of a test substance of interest in a n-octanol phase (oil phase), C0, to the concentration of the test substance in an aqueous phase, Cw (i.e., the ratio C0/Cw). In other words, the numerical value indicates which of the oil phase and the aqueous phase the ligand as the test substance is more easily dissolved in. Therefore, the larger the numerical value is, the higher the hydrophobicity of the ligand is (that is, the more poorly water-soluble the ligand is).

The octanol-water distribution coefficient can be calculated, for example, through performing measurement using a flask shaking method of JIS Z-7260-107: 2000 or an HPLC method in OECD Test Guideline 117, or through performing estimation in a computationally chemical manner based on a partial structure or constituent atoms of a substance.

In the present invention, when the found Log P value determined as the octanol-water distribution coefficient of a ligand of interest is a positive value, or when the calculated Log P value estimated as the octanol-water distribution coefficient of a ligand of interest in a computationally chemical manner is a positive value, it is determined that "the ligand is poorly water-soluble".

In a case where the octanol-water distribution coefficient is estimated in a computationally chemical manner, commercially available software can be used. For example, a numerical value (calculated Log P value) calculated using "Chemdraw Professional" manufactured by Perkinelmer, "CLOG P" manufactured by Daylight Chemical Information Systems, or the like is preferably used as the octanol-water distribution coefficient of the present invention.

The "Log S value", which is another index of poor water solubility, is an index indicating the solubility of a test substance in water. A lower Log S value indicates that a test substance, that is, a ligand, is more poorly water-soluble. As for the Log S value, for example, a value (calculated Log S value) estimated in a computationally chemical manner using commercially available software such as "Chemdraw Professional" manufactured by Perkinelmer can be used as the Log S value in the present invention.

In the above formula (1), the peptide that can be contained in R₁₄ or R₁₅ preferably has a molecular weight of 500 Da or more and 10,000 Da or less. The peptide may be, for example, a peptide containing an amino acid that does not constitute an in vivo protein, such as a D-amino acid or an amino acid in which an N-aliphatic hydrocarbon group such as an N-methyl group is modified, in view of preventing unintended decomposition or reaction of the peptide during a complex forming reaction. The peptide containing an amino acid that does not constitute an in vivo protein is generally poorly water-soluble, and a ligand to which the peptide is bonded exhibits poor water solubility as the whole ligand. In addition, such a peptide generally has peptidase resistance to thereby hardly decompose in vivo, and thus has high stability in vivo, for example, in blood; accordingly, such a peptide can be easily delivered to a target site when a radioactive metal complex containing the peptide is applied to a living body. In particular, such a peptide is preferably a cyclic peptide. Since the cyclic peptide has a chemically stronger structure than a chain peptide, the in vivo stability can be further enhanced.

The peptide that can be contained in R₁₄ or R₁₅ is not particularly limited as long as it has a molecular weight within the above range and is poorly water-soluble. Examples thereof include a straight chain peptide such as physalaemin and cyclic peptide such as daptomycin.

As described above, the reaction liquid in the complex-forming step is an aqueous reaction liquid containing water, a buffer, and a water-soluble organic solvent. As the water, distilled water or ion-exchanged water can be used, for example.

As the buffer used in the present step, one selected from the group consisting of acetic acid and a salt thereof, phosphoric acid and a salt thereof, 2-amino-2-(hydroxymethyl)propane-1,3-diol (Tris), 2-[4-(2-hydroxyethyl)-1-piperazinyl]-ethanesulfonic acid (HEPES), and a basic amino acid is preferably used. Examples of a counter ion of the buffer include cations such as an ion of an alkali metal including sodium and potassium, and a primary or quaternary ammonium including ammonium and a tetramethylammonium salt, and anions such as various halogen ions. In addition, a neutral salt such as sodium chloride may be further added. The buffer is preferably selected from these according to the types and combination of a radioactive metal nuclide and a ligand.

Among these compounds, one selected from the group consisting of acetic acid and a salt thereof, phosphoric acid and a salt thereof, Tris, HEPES, tetramethylammonium acetate, and a basic amino acid is more preferably used as the buffer. That is, in terms of a buffer solution in which buffer is dissolved in water, more preferred is a buffer solution such as an acetic acid-sodium acetate buffer solution (hereinafter, also simply referred to as an acetic acid buffer solution), an ammonium acetate buffer solution, a phosphoric acid buffer, phosphoric acid buffered saline, a Tris buffer solution, a HEPES buffer solution, or a tetramethylammonium acetate buffer solution.

The reaction liquid further contains a water-soluble organic solvent. The water-soluble organic solvent in the present step is used for increasing the solubility of a ligand in the reaction liquid to increase the amount of the ligand involved in the complex forming reaction, and is particularly suitable for increasing the solubility of a poorly water-soluble ligand. The term "water-soluble" for the water-soluble organic solvent means that when an arbitrary volume of water and an arbitrary volume of an organic solvent are mixed, the water and the organic solvent are freely mixed with no interface between the solvents observed.

As the water-soluble organic solvent, for example, a polar solvent is preferably used such as a protic solvent including methanol and ethanol, or an aprotic solvent including acetonitrile, N,N-dimethylformamide, tetrahydrofuran, dimethyl sulfoxide, and acetone. Among these solvents, at least one selected from the group consisting of acetonitrile, N,N-dimethylformamide, dimethyl sulfoxide, and ethanol is more preferably used as the water-soluble organic solvent in view of allowing the complex forming reaction to proceed satisfactorily.

In the complex-forming step, the order of adding the radioactive metal source and adding the ligand is not limited as long as a complex between the radioactive metal ion and the ligand can be formed. For example, after a mixed solvent containing water, a buffer, and a water-soluble organic solvent constituting a reaction liquid is placed in a reaction vessel in advance, one of the radioactive metal source and the ligand may be added thereto, and then the other may be added thereto to cause a reaction. Alternatively, to a solution obtained by dissolving one of the radioactive metal source and the ligand in the mixed solvent, the other may be added to cause a reaction. Alternatively, after the mixed solvent is placed in a reaction vessel in advance, the radioactive metal source and the ligand may be simultaneously added to cause a reaction.

As reaction conditions in the complex-forming step, the following conditions can be used, for example. As a reaction solvent for the present step, a mixed solvent containing water, a buffer, and a water-soluble organic solvent is used. The reaction may be performed, for example, at room temperature (25°C) or under a heating condition, and is preferably performed under a heating condition of 30°C or higher and 80°C or lower, more preferably 50°C or higher and 80°C or lower in view of both suppression of ligand decomposition and improvement in the efficiency in forming the complex. When the reaction temperature is as described above, the reaction is preferably performed for 15 minutes or more and 150 minutes or less, and more preferably 30 minutes or more and 120 minutes or less.

The amount of the reaction liquid in the present step is not particularly limited, but is practically 0.01 mL or more and 100 mL or less at the start of the present step in view of practicality in the producing step. The concentrations of the radioactive metal ion and the ligand in the reaction liquid are each independently preferably 1 µmol/L or more and 100 µmol/L or less at the start of the present step in view of increasing the yield of a target radioactive metal complex, more preferably 10 µmol/L or more and 9000 µmol/L or less, still more preferably 30 µmol/L or more and 600 µmol/L or less, and further still more preferably 50 µmol/L or more and 500 µmol/L or less. The pH of the reaction liquid can be appropriately changed depending on the physical properties of a radioactive metal, a ligand, and a buffer to be used, but is preferably 4.0 or more and 7.0 or less, more preferably 4.5 or more and 6.5 or less, and still more preferably 5.0 or more and 6.0 or less.

The obtained radioactive metal complex may be used as it is, or may be purified using a filtration filter, a membrane filter, a column packed with various fillers, chromatography, or the like.

According to the producing method of the present invention including the step described above, the solubility of the ligand in the reaction liquid can be enhanced to allow the complex forming reaction to proceed sufficiently. This makes it possible to obtain a radioactive metal complex with a high complex formation ratio. One of the features of the present invention is that a water-soluble organic solvent is contained in the reaction system. Therefore, for example, even in a case of using a poorly water-soluble ligand with which the complex forming reaction does not proceed in prior art (e.g., a ligand in which a part of the structure of the ligand exhibiting water solubility is replaced or modified to exhibit poor water solubility, or a ligand that is originally poorly water-soluble), the complex forming reaction between the radioactive metal and the ligand can proceed satisfactorily to obtain a radioactive metal complex with high yield. In particular, the present step is advantageous in that even when a radioactive metal nuclide that emits low-energy radiation difficult to detect or emits α rays is used, complex formation proceeds satisfactorily to result in high yield of the complex, and therefore the complex containing the radioactive metal nuclide can be subjected to a subsequent step in an unpurified state.

Examples of the step after the formation of the complex include the step formulating a radioactive agent containing the complex containing the radioactive metal nuclide as an active component. The formulating step can be appropriately performed by adding a pH-adjusting agent such as a citric acid buffer solution, a phosphoric acid buffer solution, or a boric acid buffer solution, a solubilizing agent such as polysorbate, a stabilizer, or an antioxidant, or by diluting with an isotonic liquid such as water or physiological saline. In addition, the formulating step may include performing sterile filtration with a membrane filter or the like thereafter to prepare an injection agent.

As the ligand used in the present invention, a ligand having any of the structures represented by the following formulas (1-a) to (1-h) is preferably used in view of making the above-described effects more remarkable. These structures can be appropriately selected depending on the type of the radioactive metal described later or the water-soluble organic solvent. The effect of the present invention is sufficiently exhibited by using a ligand having any of the structures. In the following formulas, P represents a peptide, and preferably represents a poorly water-soluble peptide having the above-described configuration. The ligand represented by each of the formulas has a poorly water-soluble peptide in a structure thereof, and the ligand as a whole thus exhibits poor water solubility.

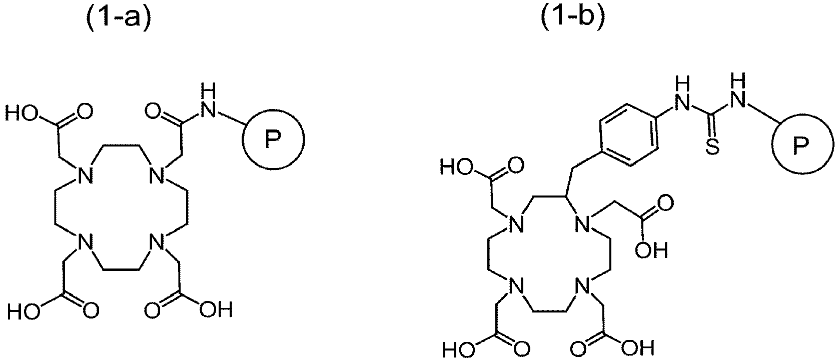

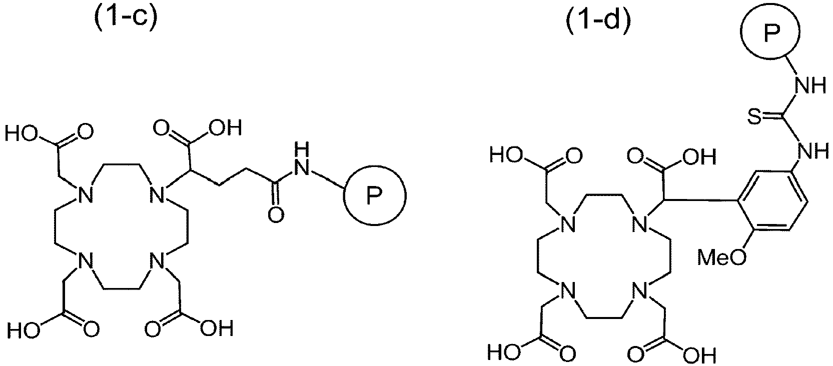

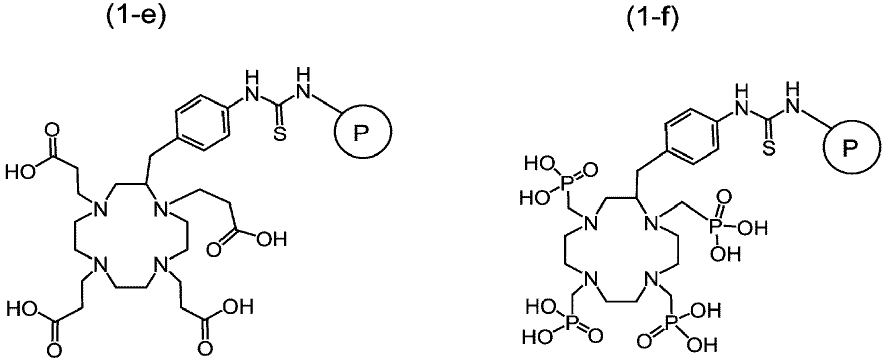

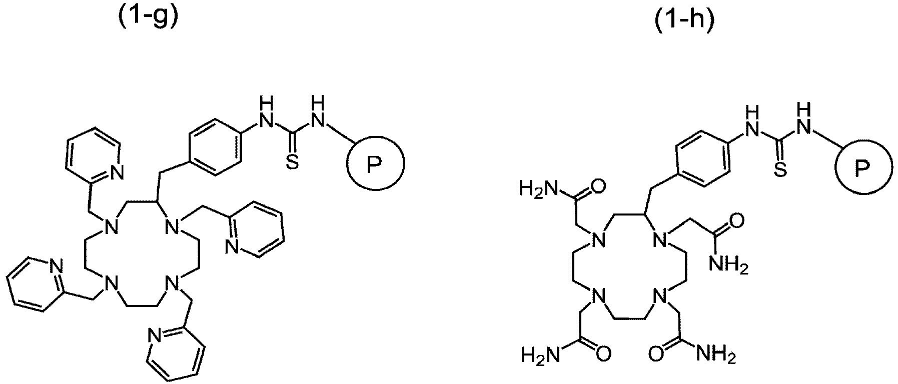

In particular, R₁₁, R₁₂, and R₁₃ more preferably each represent a carboxyalkyl group represented by -(CH₂)ₚCOOH, wherein p represents an integer of 1 or more and 3 or less, in view of achieving both ease of handling of the ligand to be used and complex stability of a radioactive metal complex to be obtained in addition to the above-described effects. In this case, preferably, one of R₁₄ and R₁₅ is a carboxyalkyl group represented by -(CH₂)ₚCOOH, wherein p represents an integer of 1 or more and 3 or less, and the other has a chemical structure containing a poorly water-soluble peptide.

The content of the water-soluble organic solvent contained in the reaction liquid is preferably 2% by volume or more, preferably 5% by volume or more and 70% by volume or less, and more preferably 5% by volume or more and 50% by volume or less, in view of achieving enhanced efficiency in forming a complex while enhancing solubility and dispersibility of the ligand in the reaction liquid.

For example, when ethanol or acetonitrile is used as the water-soluble organic solvent, the content thereof in the reaction liquid is preferably 2% by volume or more, more preferably 5% by volume or more and 70% by volume or less, still more preferably 5% by volume or more and 40% by volume or less, further still more preferably 2% by volume or more and 20% by volume or less, and further still more preferably 5% by volume or more and 15% by volume or less.

When dimethyl sulfoxide is used as the water-soluble organic solvent, the content thereof in the reaction liquid is preferably 20% by volume or more and 70% by volume or less, and more preferably 30% by volume or more and 60% by volume or less.

It is advantageous to select the type of a water-soluble organic solvent used in consideration of solubility of the ligand in the reaction liquid and, at the same time, change the content of the water-soluble organic solvent in the reaction liquid to the above-described range according to the type of a water-soluble organic solvent used. The reason for this is that the efficiency in forming the complex between the radioactive metal and the ligand can be enhanced while the ligand is appropriately dispersed or dissolved in the reaction liquid. This advantage is remarkable when a poorly water-soluble ligand is used.

The concentration of the buffer in the reaction liquid is preferably 0.05 mol/L or more and 5.0 mol/L or less, and more preferably 0.05 mol/L or more and 2.0 mol/L or less, in view of suppressing an unintended pH change during the reaction and further enhancing the efficiency in forming the complex. For example, when sodium acetate or ammonium acetate is contained as the buffer, the concentration thereof in the reaction liquid is preferably 0.05 mol/L or more and 2.0 mol/L or less, and more preferably 0.1 mol/L or more and 1 mol/L or less. When tetramethylammonium acetate is contained as the buffer, the concentration thereof in the reaction liquid is preferably 0.01 mol/L or more and 2.0 mol/L or less, and more preferably 0.1 mol/L or more and 1.0 mol/L or less.

As the radioactive metal coordinated in an ionic state in the radioactive metal complex, a metal nuclide that emits radiation of α rays, β rays, γ rays, or a combination thereof can be used. Examples of the nuclide of such a radioactive metal include a radioactive isotope of an alkali metal, an alkaline earth metal, a lanthanoid, an actinoid, a transition metal, or a metal other than these metals. Among these nuclides, ⁴⁴Sc, ⁵¹Cr, ⁵⁷Co, ⁵⁸Co, ⁶⁰Co, ⁵⁹Fe, ⁶⁷Ga, ⁶⁸Ga, ⁶⁴Cu, ⁶⁷Cu, ⁸⁹Sr, ⁸⁹Zr, ⁹⁰Y, ^{99m}Tc, ¹⁰³Ru, ¹¹¹In, ¹⁵³Sm, ¹⁶⁵Dy, ¹⁶⁶Ho, ¹⁷⁷Lu, ¹⁸⁶Re, ¹⁸⁸Re, ¹⁹⁸Au, ²⁰¹Tl, ¹⁹⁷Hg, ²⁰³Hg, ²¹²Bi, ²¹³Bi, ²¹²Pb, ²²⁷Th, or ²²⁵Ac is preferably used as the nuclide of the radioactive metal in view of being commercially available and improving the complex forming property. These radioactive metals can be produced according to a conventional method, and are preferably obtained in the form of a solution containing the radioactive metal in an ionized state.

When the radioactive metal complex is used for treating a disease, an α ray-emitting nuclide or a β⁻ ray-emitting nuclide is preferably used as the radioactive metal in view of enhancing a therapeutic effect. The α ray-emitting nuclide may be any nuclide that emits α rays in a decay process of the radioactive metal. Specifically, ²¹²Bi, ²¹³Bi, ²²⁷Th, or ²²⁵Ac is preferably used, for example. ²²⁷Th or ²²⁵Ac is more preferably used, and ²²⁵Ac is still more preferably used. The β⁻ray-emitting nuclide may be any nuclide that emits β⁻ rays in a decay process of the radioactive metal. Specifically, ⁶⁰Co, ⁵⁹Fe, ⁶⁴Cu, ⁶⁷Cu, ⁸⁹Sr, ⁹⁰Y, ^{99m}Tc, ¹⁰³Ru, ¹⁵³Sm, ¹⁶⁵Dy, ¹⁶⁶Ho, ¹⁷⁷Lu, ¹⁸⁶Re, ¹⁸⁸Re, ¹⁹⁸Au, ²⁰³Hg, ²¹²Bi, ²¹³Bi, or ²¹²Pb is preferably used, for example. ⁶⁴Cu, ⁶⁷Cu, ⁸⁹Sr, or ⁹⁰Y is more preferably used.

When the radioactive metal complex is used for the purpose of diagnosis of a disease or detection of a lesion, a β⁺ ray-emitting nuclide, an electron-capturing decay nuclide, or a γ ray-emitting nuclide is preferably used as the radioactive metal in view of enhancing diagnostic performance. The β⁺ ray-emitting nuclide may be any nuclide that emits positrons in a decay process of the radioactive metal. ⁴⁴Sc, ⁵⁸Co, ⁶⁸Ga, ⁶⁴Cu, or ⁸⁹Zr is preferably used, for example. ⁶⁴Cu or ⁸⁹Zr is more preferably used. The electron-capturing decay nuclide may be any nuclide that emits Auger electrons or characteristic X rays in a decay process of the radioactive metal. ⁵¹Cr, ⁵⁷Co, ⁵⁸Co, ⁶⁷Ga, ⁶⁸Ga, ⁶⁴Cu, ⁸⁹Zr, ¹¹¹In, ¹⁸⁶Re, ²⁰¹Tl, or ¹⁹⁷Hg is preferably used, for example. The γ ray-emitting nuclide may be any nuclide that emits γ rays by γ decay. As the nuclide that emits γ rays by γ decay, ^{99m}Tc, ⁶⁸Ga, or ²⁰¹Tl is preferably used.

For a case where the radioactive metal to be coordinated in an ionic state in the radioactive metal complex is selected on the basis of the ionic radius, examples of a radioactive metal having an ionic radius of about 70 to 130 pm include ⁶⁷Ga, ⁶⁸Ga, ⁶⁴Cu, ⁶⁷Cu, ⁸⁹Zr, ⁹⁰Y, ^{99m}Tc, ¹⁰³Ru, ¹¹¹In, ¹⁵³Sm, ¹⁶⁵Dy, ¹⁶⁶Ho, ¹⁷⁷Lu, ¹⁸⁶Re, ¹⁸⁸Re, ¹⁹⁸Au, ²⁰¹Tl, ¹⁹⁷Hg, ²⁰³Hg, ²¹²Bi, ²¹³Bi, ²¹²Pb, and ²²⁵Ac.

For example, in a case where the radioactive metal complex having ²²⁵Ac as the radioactive metal is used for the purpose of treatment of a disease, the radioactive metal complex can be suitably formed by using any of ligands having structures represented by the above formulas (1-a) to (1-h). In a case where a radioactive metal-labeled antibody having ⁸⁹Zr as the radioactive metal is used for the purpose of diagnosis of a disease or detection of a lesion, any of ligands having structures represented by the above formulas (1-b) and (1-d) to (1-h) is preferably used, and any of ligands having structures represented by the above formulas (1-b), (1-d), and (1-e) is more preferably used.

In a case where the radioactive metal complex is used for the purpose of both treatment of a disease and diagnosis of a disease or detection of a lesion, a ligand constituting the radioactive metal complex manufactured for the purpose of treatment of a disease more preferably has the same structure as a ligand constituting the radioactive metal complex manufactured for the purpose of diagnosis of a disease or detection of a lesion. That is, in this case, the radioactive metal complexes are more preferably manufactured using ligands having the same structure.

Examples of a suitable combination of the radioactive metal, the buffer, and the water-soluble organic solvent include, but are not limited to, the following combinations.
(a) A β⁺ ray-emitting nuclide is used as the radioactive metal; and the reaction liquid includes sodium acetate or ammonium acetate as the buffer in a concentration of 0.05 mol/L or more and 2.0 mol/L or less, and 20% by volume or more and 50% by volume or less of dimethyl sulfoxide as the water-soluble organic solvent. In this case, ⁸⁹Zr is more preferably used as the β⁺ ray-emitting nuclide, and a ligand having a structure represented by the above formula (1-b), (1-d), or (1-e) is more preferably used as the ligand.
(b-1) An α ray-emitting nuclide is used as the radioactive metal; and the reaction liquid includes tetramethylammonium acetate as the buffer in a concentration of 0.1 mol/L or more and 2.0 mol/L or less, and 2% by volume or more and 30% by volume or less of ethanol or acetonitrile as the water-soluble organic solvent. In this case, ²²⁵Ac is more preferably used as the α ray-emitting nuclide, and any of ligands having structures represented by the above formulas (1-a) to (1-h) is more preferably used as the ligand.

Under the condition (b-1), when ²²⁵Ac and ethanol are used as the radioactive metal and the water-soluble organic solvent, respectively, the present producing method can enhance the efficiency in forming the radioactive metal complex even if the concentration of ethanol is relatively low. In addition, this is advantageous in that the amount of the water-soluble organic solvent used can be reduced to reduce producing cost.

Under the condition (b-1), when ethanol is used as the water-soluble organic solvent, the content of ethanol in the reaction liquid is preferably 2% by volume or more and 30% by volume or less, and more preferably 2% by volume or more and 20% by volume or less.

(b-2) An α ray-emitting nuclide is used as the radioactive metal; and the reaction liquid includes sodium acetate or ammonium acetate as the buffer in a concentration of 0.05 mol/L or more and 2.0 mol/L or less, and 2% by volume or more and 30% by volume or less of ethanol or acetonitrile as the water-soluble organic solvent. In this case, ²²⁵Ac is more preferably used as the α ray-emitting nuclide, and any of ligands having structures represented by the above formulas (1-a) to (1-h) is more preferably used as the ligand.

Under the condition (b-2), when ²²⁵Ac and ethanol are used as the radioactive metal and the water-soluble organic solvent, respectively, the present producing method can enhance the efficiency in forming the radioactive metal complex, even if the concentration of ethanol is relatively low and/or even if the concentration of the ligand is high. This is advantageous in that the amount of the water-soluble organic solvent used can be reduced to reduce producing cost, and also advantageous in that even when a large amount of the ligand is used in commercially producing the radioactive metal complex, the high efficiency in forming the radioactive metal complex can be achieved while the solubility of the ligand in the reaction liquid is maintained.

(b-3) An α ray-emitting nuclide is used as the radioactive metal; and the reaction liquid includes sodium acetate or ammonium acetate as the buffer in a concentration of 0.05 mol/L or more and 2.0 mol/L or less, and 10% by volume or more and 50% by volume or less of dimethyl sulfoxide as the water-soluble organic solvent. In this case, ²²⁵Ac is more preferably used as the α ray-emitting nuclide, and any of ligands having structures represented by the above formulas (1-a) to (1-h) is more preferably used as the ligand.

Under the condition (b-3), when ²²⁵Ac and dimethyl sulfoxide are used as the radioactive metal and the water-soluble organic solvent, respectively, the present producing method can enhance the efficiency in forming the radioactive metal complex even if the concentration of the ligand is high. This is advantageous in that even when a large amount of the ligand is used in commercially producing the radioactive metal complex, the high efficiency in forming the radioactive metal complex can be achieved while the solubility of the ligand in the reaction liquid is maintained.

The peptide that can be used in the present invention can be synthesized by a method such as a liquid phase synthesis method, a solid phase synthesis method, an automatic peptide synthesis method, a gene recombination method, a phage display method, genetic code reprogramming, or a random non-standard peptide integrated discovery (RaPID) method. In the synthesis of the peptide, a functional group of an amino acid used may be protected as necessary.

When a ligand containing a poorly water-soluble peptide in a structure thereof is used as the ligand, the poorly water-soluble peptide and a ligand precursor are preferably linked to each other by an amide bond or a thiourea bond to form a poorly water-soluble ligand. The amide bond can be formed, for example, by allowing an amino group from a side chain of an amino acid constituting the peptide to react with a carboxy group of the ligand precursor. Examples of such a ligand include a ligand having a structure represented by the above formula (1-a) or (1-c).

The thiourea bond can be formed, for example, by allowing an amino group from a side chain of an amino acid constituting the peptide to react with an isothiocyanate group of the ligand precursor, or by allowing a thiol group from a side chain of an amino acid constituting the peptide to react with a maleimide group of the ligand precursor. Examples of such a ligand include a ligand having a structure represented by any of the above formulas (1-b) and (1-d) to (1-h).

### Examples

Hereinafter, the present invention will be described in more detail with reference to Examples. However, the scope of the present invention is not limited to Examples below.

Examples 1-1 to 1-4: study of ⁸⁹Zr labeling (type of organic solvent)

### Example 1-1

⁸⁹Zr was used as a radioactive metal element. DOTA (in the formula (1), R₁₁, R₁₂, R₁₃, and R₁₄ each represent a "-CH₂COOH" group, and R₁₅ represents a hydrogen atom) was used as a ligand.

The ligand was dissolved in water containing 90% by volume of dimethyl sulfoxide as an organic solvent to prepare a solution containing the ligand in a concentration of 200 µmol/L. 0.029 mL of this solution, 0.02 mL of a solution containing ⁸⁹Zr ion (solvent: 0.1 mol/L hydrochloric acid aqueous solution, radioactivity concentration: 33.4 MBq/mL) as a radioactive metal source, and 0.01 mL of a 1.5 mol/L acetic acid buffer solution (pH 5.5) were mixed to obtain a reaction liquid, and the reaction liquid was allowed to react under heating conditions to obtain a ⁸⁹Zr complex solution. The heating temperature of the reaction liquid was 70°C, and the heating time was 60 minutes. Using thin layer chromatography (manufactured by Merck, model number: 1.15685.0001, developing solvent: 10 vol% ammonium chloride aqueous solution/methanol (1:1)), the percentage of the radioactivity count of the ⁸⁹Zr complex with respect to the radioactivity count of the total ⁸⁹Zr including ⁸⁹Zr that had not reacted was determined as a labeling ratio. The labeling ratio of the ⁸⁹Zr complex in the present Example was 84%.

### Example 1-2

An experiment was performed under the same conditions as in Example 1-1, except that DOTA used as a ligand was dissolved in water containing 90% by volume of acetonitrile as an organic solvent. The labeling ratio of the ⁸⁹Zr complex was 59%.

### Example 1-3

An experiment was performed under the same conditions as in Example 1-1, except that DOTA used as a ligand was dissolved in water containing 90% by volume of ethanol as an organic solvent. The labeling ratio of the ⁸⁹Zr complex was 55%.

### Example 1-4

An experiment was performed under the same conditions as in Example 1-1, except that DOTA used as a ligand was dissolved in water containing 90% by volume of N,N-dimethylformaldehyde as an organic solvent. The labeling ratio of the ⁸⁹Zr complex was 54%.

Examples 2-1 to 2-6: study of ⁸⁹Zr labeling (concentration of buffer)

### Example 2-1

DOTA was used as a ligand, and the ligand was dissolved in a 1.5 mol/L acetic acid buffer solution (pH 5.5) containing 90% by volume of dimethyl sulfoxide as an organic solvent to prepare a solution containing the ligand in a concentration of 200 µmol/L. 0.029 mL of this solution, 0.02 mL of a solution containing ⁸⁹Zr ion (solvent: 0.1 mol/L hydrochloric acid aqueous solution, radioactivity concentration: 25.2 MBq/mL) as a radioactive metal source, and 0.01 mL of a 1.5 mol/L acetic acid buffer solution (pH 5.5) were mixed to obtain a reaction liquid, and the reaction liquid was allowed to react under heating conditions to obtain a ⁸⁹Zr complex solution. The final concentration of the buffer in the reaction liquid was 0.33 mol/L. The heating temperature of the reaction liquid was 70°C, and the heating time was 15 minutes. Thin layer chromatography was performed under the same conditions as in Example 1. The labeling ratio of the ⁸⁹Zr complex was 60%.

### Example 2-2

An experiment was performed under the same conditions as in Example 2-1, except that DOTA used as a ligand was dissolved in water containing 90% by volume of dimethyl sulfoxide as an organic solvent to prepare a solution containing the ligand in a concentration of 200 µmol/L. The final concentration of the buffer in the reaction liquid was 0.25 mol/L. The labeling ratio of the ⁸⁹Zr complex was 55%.

### Example 2-3

DOTA was used as a ligand, and the ligand was dissolved in water containing 90% by volume of dimethyl sulfoxide as an organic solvent to prepare a solution containing the ligand in a concentration of 200 µmol/L. An experiment was performed under the same conditions as in Example 2-1, except that 0.029 mL of this solution, 0.02 mL of a solution containing ⁸⁹Zr ion (solvent: 0.1 mol/L hydrochloric acid aqueous solution, radioactivity concentration: 25.2 MBq/mL) as a radioactive metal source, and 0.01 mL of a 0.75 mol/L acetic acid buffer solution (pH 5.5) were mixed to obtain a reaction liquid, and that the reaction liquid was allowed to react under heating conditions. The final concentration of the buffer in the reaction liquid was 0.13 mol/L. The labeling ratio of the ⁸⁹Zr complex was 66%.

### Example 2-4

An experiment was performed under the same conditions as in Example 2-1 to obtain a ⁸⁹Zr complex solution, except that DOTA used as a ligand was dissolved in water to prepare a solution containing the ligand in a concentration of 200 µmol/L. The final concentration of the buffer in the reaction liquid was 0.25 mol/L. The labeling ratio of the ⁸⁹Zr complex was 50%.

### Example 2-5

An experiment was performed under the same conditions as in Example 2-1 to obtain a ⁸⁹Zr complex solution, except that DOTA used as a ligand was dissolved in a 1.5 mol/L acetic acid buffer solution (pH 5.5) to prepare a solution containing the ligand in a concentration of 200 µmol/L. The final concentration of the buffer in the reaction liquid was 1.00 mol/L. The labeling ratio of the ⁸⁹Zr complex was 28%.

### Example 2-6

An experiment was performed under the same conditions as in Example 2-1 to obtain a ⁸⁹Zr complex solution, except that DOTA used as a ligand was dissolved in a 3.0 mol/L acetic acid buffer solution (pH 5.5) to prepare a solution containing the ligand in a concentration of 200 µmol/L. The labeling ratio of the ⁸⁹Zr complex was 10%.

Examples 3-1 to 3-4: study of ⁸⁹Zr labeling (concentration of ligand)

### Example 3-1

DOTA was used as a ligand, and the ligand was dissolved in water containing 90% by volume of dimethyl sulfoxide as an organic solvent to prepare a solution containing the ligand in a concentration of 200 µmol/L. 0.029 mL of this solution, 0.02 mL of a solution containing ⁸⁹Zr ion (solvent: 0.1 mol/L hydrochloric acid aqueous solution, radioactivity concentration: 28.5 MBq/mL) as a radioactive metal source, and 0.01 mL of a 1.5 mol/L acetic acid buffer solution (pH 5.5) were mixed to obtain a reaction liquid, and the reaction liquid was allowed to react under heating conditions to obtain a ⁸⁹Zr complex solution. The final concentration of the ligand in the reaction liquid was 100 µmol/L. The heating temperature of the reaction liquid was 70°C, and the heating time was 60 minutes. Thin layer chromatography was performed under the same conditions as in Example 1-1. The labeling ratio of the ⁸⁹Zr complex was 89%.

### Example 3-2

An experiment was performed under the same conditions as in Example 3-1 to obtain a ⁸⁹Zr complex solution, except that DOTA used as a ligand was dissolved in water containing 90% by volume of dimethyl sulfoxide as an organic solvent such that the final concentration of the ligand in the reaction liquid was 50 µmol/L. The labeling ratio of the ⁸⁹Zr complex was 50%.

### Example 3-3

An experiment was performed under the same conditions as in Example 3-1 to obtain a ⁸⁹Zr complex solution, except that DOTA used as a ligand was dissolved in water containing 90% by volume of dimethyl sulfoxide as an organic solvent such that the final concentration of the ligand in the reaction liquid was 10 µmol/L. The labeling ratio of the ⁸⁹Zr complex was 12%.

### Example 3-4

An experiment was performed under the same conditions as in Example 3-1 to obtain a ⁸⁹Zr complex solution, except that DOTA used as a ligand was dissolved in water containing 90% by volume of dimethyl sulfoxide as an organic solvent such that the final concentration of the ligand in the reaction liquid was 1 µmol/L. The labeling ratio of the ⁸⁹Zr complex was 9%.

Examples 4-1 to 4-6: study of ²²⁵Ac labeling (type and concentration of organic solvent)

### Example 4-1

DOTA was used as a ligand, and the ligand was dissolved in water containing 10% by volume of ethanol as an organic solvent to prepare a solution containing the ligand in a concentration of 100 µmol/L. 0.039 mL of this solution, 0.02 mL of a solution containing ²²⁵Ac ions (solvent: 0.2 mol/L hydrochloric acid aqueous solution, radioactivity concentration: 5 MBq/mL) as a radioactive metal source, and 0.016 mL of a 0.5 mol/L tetramethylammonium acetate buffer solution (pH 7.8) were mixed to obtain a reaction liquid, and the reaction liquid was allowed to react under heating conditions to obtain an ²²⁵Ac complex solution. The heating temperature of the reaction liquid was 70°C, and the heating time was 60 minutes. Thin layer chromatography was performed under the same conditions as in Example 1-1. The labeling ratio of the ²²⁵Ac complex was 83%.

### Example 4-2

An experiment was performed under the same conditions as in Example 4-1 to obtain an ²²⁵Ac complex solution, except that DOTA used as a ligand was dissolved in water containing 10% by volume of acetonitrile as an organic solvent. The labeling ratio of the ²²⁵Ac complex was 86%.

### Examples 4-3 and 4-4

An experiment was performed under the same conditions as in Example 4-1 to obtain an ²²⁵Ac complex solution, except that DOTA used as a ligand was dissolved in water containing 90% by volume or 50% by volume of ethanol as an organic solvent to prepare a solution containing the ligand in a concentration of 100 µmol/L. The labeling ratios of the ²²⁵Ac complex were 25% and 67%, respectively.

### Examples 4-5 and 4-6

An experiment was performed under the same conditions as in Example 4-1 to obtain an ²²⁵Ac complex solution, except that the ligand was dissolved in water containing 90% by volume or 50% by volume of acetonitrile as an organic solvent to prepare a solution containing the ligand in a concentration of 100 µmol/L. The labeling ratios of the ²²⁵Ac complex were 27% and 69%, respectively.

### Comparative Example 1

An experiment was performed under the same conditions as in Example 1-1, except that DOTA used as a ligand was dissolved in a 0.5 mol/L phosphoric acid buffer solution (pH 5.5) to prepare a solution containing the ligand in a concentration of 2 mmol/L. In the present Comparative Example, the reaction liquid did not contain any water-soluble organic solvent. The labeling ratio of the ⁸⁹Zr complex was 0%, and the complex forming reaction did not proceed at all.

### Examples 5-1 and 5-2

A reaction is caused under the same conditions as in Example 1, except that a ligand having DOTA and a peptide in the structure thereof is used. The peptide has a calculated negative Log S value as estimated in a computationally chemical manner, and the ligand has a calculated negative Log S value as the whole ligand. In this case, the complex forming reaction proceeds to obtain a ⁸⁹Zr complex solution.

Specifically, in the present Example, a ligand was used that was obtained by bonding p-SCN-Bn-DOTA and, as a peptide, physalaemin (Example 5-1; molecular weight: 1265 Da, calculated Log S value: -6.664) or daptomycin (Example 5-2; molecular weight: 1619 Da, calculated Log S value: -9.777) to each other by a conventional method. Each of these ligands has a structure represented by the formula (1-b), and has a structure derived from DOTA and a peptide in a structure thereof. Details of the chemical structure are indicated in the following formulas (E1) and (E2). Each of these ligands has a calculated negative Log S value, and is therefore poorly water-soluble.

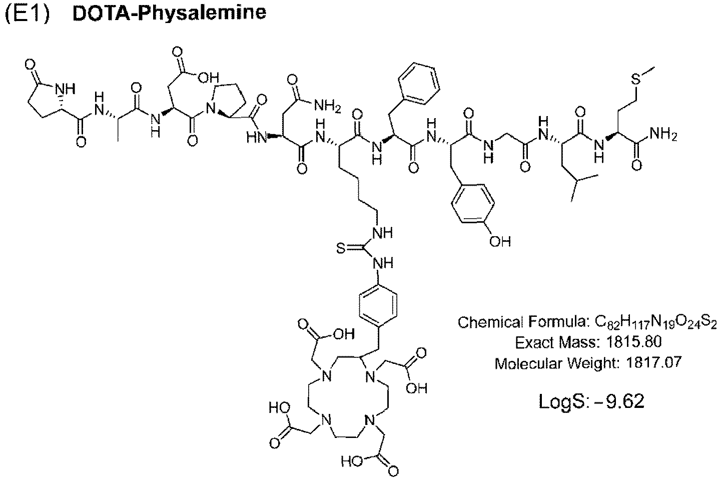

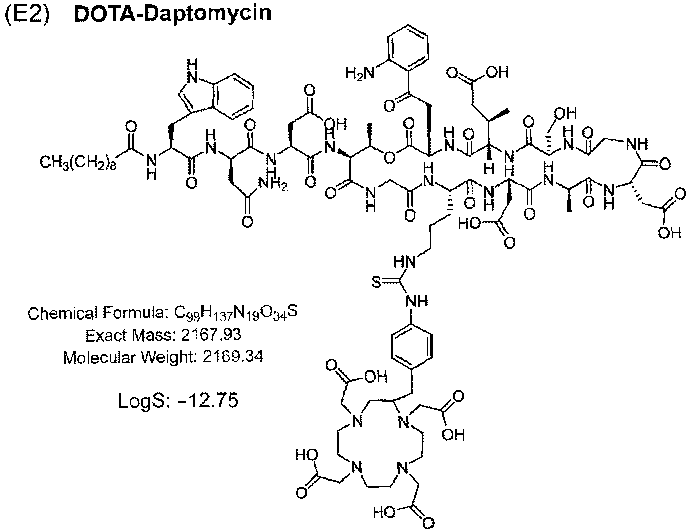

The details of the producing method in the present Example are as follows. First, the ligand was dissolved in a 1.5 mol/L acetic acid buffer solution (pH 5.5) containing 45% by volume of dimethyl sulfoxide (DMSO) as a water-soluble organic solvent to prepare a solution. This solution, a solution containing ⁸⁹Zr ions (solvent: 0.1 mol/L hydrochloric acid aqueous solution, radioactivity concentration: 33.4 MBq/mL) as a radioactive metal source, and a 1.5 mol/L acetic acid buffer solution (pH 5.5) were mixed to obtain a reaction liquid, and 59 µL of the reaction liquid was allowed to react under heating conditions of 70°C for two hours to obtain a ⁸⁹Zr complex solution. The ligand concentration and the amount of radioactivity of the reaction liquid at the start of the reaction were as shown in Table 1 below.

Using thin layer chromatography (iTLC-SG manufactured by Agilent Technologies, developing solvent: water/acetonitrile (1:1)), the percentage of the radioactivity count of the ⁸⁹Zr complex with respect to the radioactivity count of the total ⁸⁹Zr including ⁸⁹Zr that had not reacted was determined as a labeling ratio for the obtained ⁸⁹Zr complex. The results of the labeling ratio of the ⁸⁹Zr complex are shown in Table 1 below.

**[Table 1]**

| | Ligand | Ligand concentration [µmοl/L] | Amount of ⁸⁹Zr radioactivity [MBq] | Buffer | Water-soluble organic solvent | Labeling ratio of ⁸⁹Zr complex [%] |
|---|---|---|---|---|---|---|
| Example 5-1 | DOTA-Physalaemin | 450 | 6.08 | 0.25 mol/L acetic acid-sodium acetate buffer solution (pH 5.5) | 45% by volume DMSO | 84.0 |
| Example 5-2 | DOTA-Daptomycin | | 5.84 | | | 96.8 |

### Comparative Example 2

A reaction is caused under the same reaction conditions as in Example 5 except that the reaction liquid does not contain any water-soluble organic solvent. In this case, the complex forming reaction does not proceed.

### Examples 6-1 and 6-2

The ligands represented by the formulas (E1) and (E2) were used. The ligands was dissolved in water containing ethanol as an organic solvent to prepare a solution. This solution, a solution containing ²²⁵Ac ions (solvent: 0.2 mol/L hydrochloric acid aqueous solution, radioactivity concentration: 5 MBq/mL) as a radioactive metal source, and a 0.5 mol/L tetramethylammonium acetate buffer solution (pH 7.8) were mixed to obtain a reaction liquid, and 79 µL of the reaction liquid was allowed to react under heating conditions of 70°C for one hour to obtain an ²²⁵Ac complex solution. The ligand concentration and the amount of radioactivity of the reaction liquid at the start of the reaction were as shown in Table 2 below. The concentration of the water-soluble organic solvent (ethanol) in the reaction liquid was 10% by volume.

Thin layer chromatography was performed under the same conditions as in Example 5-1. The results of the labeling ratio (%) of the ²²⁵Ac complex are shown in Table 2 below.

**[Table 2]**

| | Ligand | Ligand concentration [µmοl/L] | Amount of ²²⁵Ac radioactivity [kBq] | Buffer | Water-soluble organic solvent | Labeling ratio of ²²⁵Ac complex [%] |
|---|---|---|---|---|---|---|
| Example 6-1 | DOTA-Physalaemin | 500 | 358 | 0.1 mol/L tetramethylammonium acetate buffer solution (pH 7.8) | 10% by volume ethanol | 91.2 |
| Example 6-2 | DOTA -Daptomycin | | 359 | | | 97.4 |

### Examples 7-1 to 7-4

In the present Examples, the ligand represented by the formula (E2) was used. The ligand concentration and the amount of ²²⁵Ac radioactivity of the reaction liquid at the start of the reaction were as shown in Table 3 below. In addition, the type and concentration of the water-soluble organic solvent in the reaction liquid were changed as shown in Table 3 below. A reaction was caused under the same reaction conditions as in Example 6-1 except for the above, to thereby obtain an ²²⁵Ac complex solution. The results of the labeling ratio (%) of the ²²⁵Ac complex are shown in Table 3 below.

### Examples 7-5 to 7-11

In the present Examples, the ligand represented by the formula (E2) was used. The ligand concentration and the amount of ²²⁵Ac radioactivity of the reaction liquid at the start of the reaction were as shown in Table 3 below. In addition, the type of the buffer in the reaction liquid and the type and concentration of the water-soluble organic solvent in the reaction liquid were changed as shown in Table 3 below. A reaction was caused under the same reaction conditions as in Example 6-1 except for the above, to thereby obtain an ²²⁵Ac complex solution. The results of the labeling ratio (%) of the ²²⁵Ac complex are shown in Table 3 below.

**[Table 3]**

| | Ligand | Ligand concentration [µmol/L] | Amount of ²²⁵Ac radioactivity [kBq] | Buffer | Water-soluble organic solvent | Concentration of water-soluble organic solvent in reaction liquid [vol%] | Labeling ratio of ²²⁵Ac complex [%] |
|---|---|---|---|---|---|---|---|
| Example 7-1 | DOTA Daptomycin | 500 | 176 | 0.1 mol/L tetramethylammonium acetate buffer solution (pH 7.8) | Ethanol | 2 | 96.8 |
| Example 7-2 | | | 216 | | | 5 | 96.5 |
| Example 7-3 | | | 165 | | | 15 | 96.6 |
| Example 7-4 | | | 276 | | | 20 | 95.7 |
| Example 7-5 | | | 91 | 0.1 mol/L acetic acid-sodium acetate buffer solution (pH 5.5) | | 2 | 98.8 |
| Example 7-6 | | | 76 | | | 5 | 98.1 |
| Example 7-7 | | | 76 | | | 15 | 98.0 |
| Example 7-8 | | | 59 | | | 20 | 96.7 |
| Example 7-9 | | | 76 | | DMSO | 10 | 98.4 |
| Example 7-10 | | | 79 | | | 30 | 99.5 |
| Example 7-11 | | | 76 | | | 50 | 99.7 |

It is found from the above that, when the water-soluble organic solvent is used in the reaction liquid, the complex forming reaction proceeds satisfactorily. In addition, it is found that the complex forming reaction proceeds satisfactorily by adjusting the concentrations of the water-soluble organic solvent and the buffer or the concentration of the ligand to an appropriate concentration range according to the type of the water-soluble organic solvent or the buffer.

It is found that under the producing condition that ⁸⁹Zr and a poorly water-soluble ligand are used, the complex forming ratio (labeling ratio) is further improved by using a combination of DMSO in a predetermined concentration and an acetic acid buffer solution.

It is found that under the producing conditions that ²²⁵Ac and a poorly water-soluble ligand are used, the complex forming ratio (labeling ratio) is further improved by using a combination of ethanol at a predetermined concentration and an acetic acid buffer solution or a tetramethylammonium acetate buffer solution or by using a combination of DMSO at a predetermined concentration and an acetic acid buffer solution.

Thus, the producing method of the present invention is excellent in the efficiency in forming a complex, and an effect thereof is remarkable particularly when a poorly water-soluble ligand is used.

## Claims

1. A method for producing a radioactive metal complex, the method comprising a step of allowing a radioactive metal to react with a ligand represented by the following formula (1) in a reaction liquid to form a radioactive metal complex, wherein
the reaction liquid contains water, a buffer, and a water-soluble organic solvent, and
the radioactive metal is ⁸⁹Zr or ²²⁵Ac, 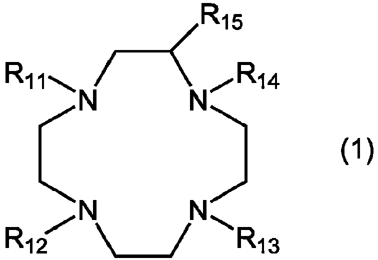
wherein R₁₁, R₁₂, and R₁₃ each independently represent a group of -(CH₂)ₚCOOH, - (CH₂)ₚC₅H₅N, -(CH₂)ₚPO₃H₂, or -(CH₂)ₚCONH₂; one of R₁₄ and R₁₅ represents a hydrogen atom or a group of -(CH₂)ₚCOOH, -(CH₂)ₚC₅H₅N, -(CH₂)ₚPO₃H₂, -(CH₂)ₚCONH₂, or - (CHCOOH)(CH₂)ₚCOOH, and the other represents a group of -(CH₂)ₚCOOH, -(CH₂)ₚC₅H₅N, - (CH₂)ₚPO₃H₂, or -(CH₂)ₚCONH₂, or a group linked to a peptide; and p represents an integer of 0 or more and 3 or less.

2. The method for producing a radioactive metal complex according to claim 1, wherein the ligand is a poorly water-soluble ligand.

3. The method for producing a radioactive metal complex according to claim 1 or 2, wherein
in the above formula, R₁₁, R₁₂, and R₁₃ each represent a group of -(CH₂)ₚCOOH; and one of R₁₄ and R₁₅ represents a hydrogen atom or a group of -(CH₂)ₚCOOH, and the other represents a group of -(CH₂)ₚCOOH or a group linked to a peptide,
when R₁₄ represents a group linked to a peptide, R₁₅ represents a hydrogen atom, and
when R₁₄ does not represent a group linked to a peptide, R₁₅ represents a group linked to a peptide.

4. The method for producing a radioactive metal complex according to any one of claims 1 to 3, wherein the reaction liquid has a content of the water-soluble organic solvent of 2% by volume or more and 50% by volume or less.

5. The method for producing a radioactive metal complex according to any one of claims 1 to 4, wherein the water-soluble organic solvent is a polar solvent.

6. The method for producing a radioactive metal complex according to any one of claims 1 to 6, wherein the water-soluble organic solvent is at least one selected from the group consisting of acetonitrile, N,N-dimethylformamide, dimethyl sulfoxide, and ethanol.

7. The method for producing a radioactive metal complex according to claim 6, wherein the reaction liquid contains 20% by volume or more and 50% by volume or less of dimethyl sulfoxide as the water-soluble organic solvent.

8. The method for producing a radioactive metal complex according to claim 6 or 7, wherein the reaction liquid contains 2% by volume or more and 50% by volume or less of ethanol or acetonitrile as the water-soluble organic solvent.

9. The method for producing a radioactive metal complex according to any one of claims 1 to 8, wherein the buffer is one selected from the group consisting of acetic acid and a salt thereof, phosphoric acid and a salt thereof, 2-amino-2-(hydroxymethyl)propane-1,3-diol, 2-[4-(2-hydroxyethyl)-1-piperazinyl]-ethanesulfonic acid, tetramethylammonium acetate, and a basic amino acid.

10. The method for producing a radioactive metal complex according to claim 9, wherein the buffer is contained in a concentration of 0.01 mol/L or more and 5.0 mol/L or less in the reaction liquid.

11. The method for producing a radioactive metal complex according to claim 10, wherein sodium acetate or ammonium acetate as the buffer is contained in a concentration of 0.05 mol/L or more and 2.0 mol/L or less in the reaction liquid.

12. The method for producing a radioactive metal complex according to claim 10, wherein tetramethylammonium acetate as the buffer is contained in a concentration of 0.1 mol/L or more and 2.0 mol/L or less in the reaction liquid.

13. The method for producing a radioactive metal complex according to any one of claims 1 to 12, wherein the radioactive metal is allowed to react with the ligand in the reaction liquid at 30°C or higher and 80°C or lower.

14. The method for producing a radioactive metal complex according to any one of claims 1 to 13, wherein the peptide has a molecular weight of 500 Da or more and 10,000 Da or less.
